(19) Europäisches Patentamt
European Patent Office
Office européen des brevets

(11) EP 0 987 268 B1

(12) EUROPEAN PATENT SPECIFICATION

(45) Date of publication and mention of the grant of the patent:
**27.03.2002 Bulletin 2002/13**

(51) Int Cl.[7]: **C07D 493/04**, A61K 31/425 // (C07D493/04, 313:00, 303:00)

(21) Application number: **98115894.2**

(22) Date of filing: **24.08.1998**

# (54) Pharmaceutical agents containing epothilone A-N-oxide and/or epothilone B-N-oxide

Epothilon A N-Oxid und/oder Epothilon B N-Oxid enthaltende pharmazeutische Agentien

Agents pharmaceutiques contenant le N-oxyde d'épothilone A et/ou le N-oxyde d'épothilone B

(84) Designated Contracting States:
**AT BE CH CY DE DK ES FI FR GB GR IE IT LI LU MC NL PT SE**
Designated Extension States:
**AL LT LV MK RO SI**

(30) Priority: **05.08.1998 EP 98114736**

(43) Date of publication of application:
**22.03.2000 Bulletin 2000/12**

(73) Proprietor: **Gesellschaft für Biotechnologische Forschung mbH (GBF)**
**38124 Braunschweig (DE)**

(72) Inventors:
- **Höfle, Gerhard, Dr.**
  **38124 Braunschweig (DE)**
- **Glaser, Nicole**
  **38124 Braunschweig (DE)**
- **Leibold, Thomas, Dr.**
  **38124 Braunschweig (DE)**

(74) Representative: **Boeters, Hans Dietrich, Dr. et al**
**Patentanwälte Boeters & Bauer,**
**Bereiteranger 15**
**81541 München (DE)**

(56) References cited:
**WO-A-93/10121**
**WO-A-98/38192**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 987 268 B1

## Description

**[0001]** The invention concerns a pharmaceutical agent being active against multidrug resistant cell lines and comprising epothilone A-N-oxide of the following formula:

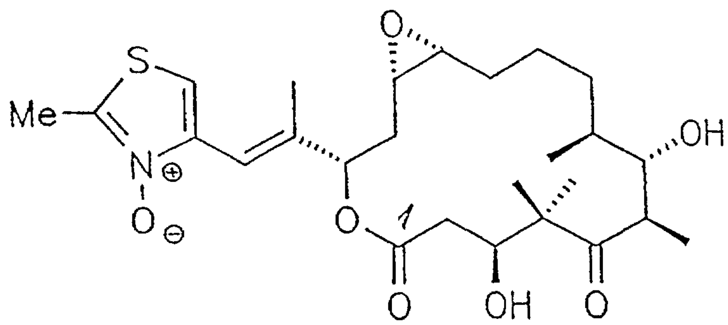

and/or epothilone B-N-oxide of the following formula:

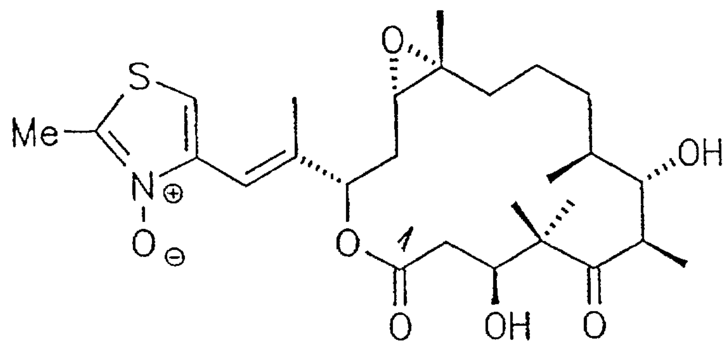

as active ingredient facultatively in the presence of usual auxiliary agents, carriers and/or diluents.

**[0002]** As regards epothilone A and epothilone B reference is made to DE-A-41 38 042.

**[0003]** **Epothilone A N-oxide:** 100 mg of 70% *m*-chloroperbenzoic acid in 0.5 ml of dichloromethane were added to 100 mg of epothilone A in 1 ml of dichloromethane. After the mixture has been stirred for 6 hours at room temperature, it is diluted with dichloromethane and extracted by shaking in succession with sodium sulphite solution to destroy excess peracid and with sodium bicarbonate solution. The solvent is evaporated in vacuo, and the residue is separated by preparative HPLC on a Nucleosil RP-18 column (250 $\times$ 20 mm, eluent methanol/water 60:40). Yield 60 mg of colourless oil.

$R_f$=0.60 (silica gel TLC aluminium foil, eluent dichloromethane/methanol 9:1);
ESI-MS (neg. ions) m/z 510;
UV (methanol): lamda max. 240 nm;
$^{13}$C NMR ($CDCl_3$): C-1 170.5, C-2 39.9, C-3 70.8, C-4 55.1, C-5 221.4, C-6 40.9, C-7 72.9, C-8 37.6, C-9 31.8, C-10 22.8, C-11 28.0, C-12 58.0, C-13 55.8, C-14 32.2, C-15 75.5, C-16 144.5, C-17 111.4, C-18 143.4, C-19 110.3, C-20 145.6, C-21 13.5, C-22 15.4, C-23 23.3, C-24 12.0, C-25 16.5, C-27 18.2 ppm;
$^1$H NMR ($CDCl_3$): 2a-H 2.12 dd, 2b-H 2.47 dd, 3-H 4.55 dd, 3-OH 6.48 broad, 6-H 3.25 dq, 7-H 3.72 dd, 8-H 1.81 m, 9a-H 1.34 m, 9b-H 1.56 m, 10-$H_2$ 1.48 m, 11a-H 1.27 m, 11b-H 1.87 m, 12-H 2.92 ddd, 13-H 2.98 m, 14a-H 1.67 ddd, 14b-H 2.23 d, 15-H 5.33 d, 17-H 6.82 s, 19-H 7.09 s, 21-$H_3$ 2.61 s, 22-$H_3$ 1.02 s, 23-$H_3$ 1.42 s, 24-$H_3$ 1.18 d, 25-$H_3$ 0.99 d, 27-$H_3$ 2.04 s ppm.

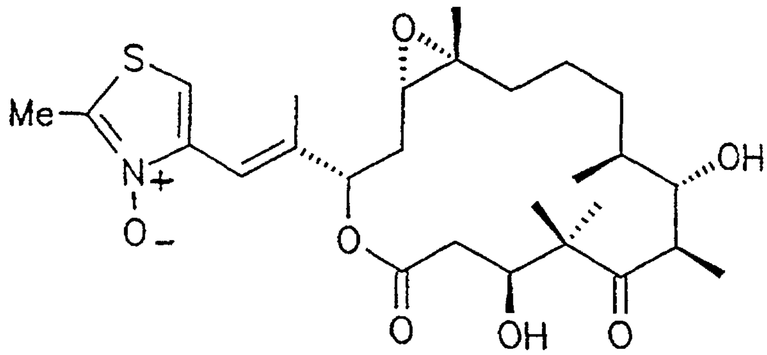

**Epothilone B - *N*- oxide**

**[0004]** To a solution of 2 mmol of *m*-chloroperbenzoic acid in 1 1 ml dichloromethane were added 507 mg (1 mmol) of epothilone B and stirred for 3 h at room temperature. Ethyl acetate was added and excess of perbenzoic acid was reduced with an aquous solution of sodium sulfite. The organic layer was dried with magnesium sulfate and evaporated to give 0.6 g of crude product containing 390 mg (75%) of the desired product according to HPLC analysis. Separation was achieved by chromatography on a HD-SIL C-18, 35-70 µm column (9 cm ϕ, length 83 cm) with the solvent system 45% acetonitrile/55 % ammonium acetate buffer 50 mM, pH 7.5; detection 254 nm. Yield 207 mg.

DC:$\underline{R}_f$=0.19 (silica-gel Si60, dichloromethane/methanol 95:5, detection with vanillin/-sulfuric acid, blue-gray coloration on heating to 120°C);
HPLC:$\underline{R}_t$ = 4.1 min (Nucleosil, C-18, 7 µm, 250 x 4 mm column, solvent system methanol/water 70:30, 1 ml/min, detection at 254 nm);
UV (MeOH): $\lambda_{max}$ (ε) = 283 sh (log ε 3.22), 264 sh (3.63) 236 nm(4.19);
IR (KBr): $\bar{\nu}$ = 3439, 2963, 2936, 2877, 1740, 1689 $cm^{-1}$;
$^1$H-NMR ($CDCl_3$) : δ = 2.13 (dd, J = 12.9, 12.0, 2-Ha); 2.47 (t=12.0, 2-Hb); 4.58 (m, 3-H); 3.29 (dq, J = 2.0, 5.8, 6-H); 3.70 (d, J = 5.8, 7-H); 1.20 - 1.90 (m, 8-H, 9-$H_2$, 10-$H_2$, 11-$H_2$); 2.75 (m, 13-H); 1.66 (m, 14-Ha); 2.23 (dbr, J = 15.6, 14-Hb); 5.32 (d, J = 11.6. 15-H); 6.79 (sbr, 17-H); 7.08 (s, 19-H); 2.60 (s, 21-$H_3$); 1.02 (s, 22-$H_3$); 1.27 (s, 23-$H_3$); 1.16 (d, J = 6.8, 24-$H_3$); 0.99 (d, J = 7.1, 25-$H_3$); 1.41 (s, 26-$H_3$); 2.07 (s, 27-$H_3$);
$^{13}$C-NMR (150 MHz, $CDCl_3$): δ = 170.5 (C-1), 40.0 (C-2), 70.9 (C-3), 55.1 (C-4), 221.4 (C-5), 40.7 (C-6), 72.5 (C-7), 37.3 (C-8), 31.5 (C-9), a 22.0 (C-10), 33.3 (C-11), b 62.3 (C-12), 63.0 (C-13), 33.4 (C-14), 75.6 (C-15), 144.3 (C-16), 111.1 (C-17), 143.3 (C-18), 110.3 (C-19), 144.7 (C-20), 13.4 (C-21), 15.3 (C-22), 23.2 (C-23), 12.5 (C-24), 16.5 (C-25), 22.2 (C-26), 18.2 (C-27);
[a] EI-MS (70 eV) : $\underline{m}/\underline{z}$ (%) 523.2604 (40 $M^+$), ber. ($C_{27}H_{41}NO_7S$) 523.2629, 424 (38), 336 (42), 194(18) 182(75), 154(100), 126(42).

a and b can be exchanged

| **Cytotoxic activity of epothilone B-*N*-oxide** | |
|---|---|
| Cell line | $IC_{50}$[nM] |
| mouse fibroblast L929 (ATCC CCL1) | 4 |
| human cervix carcinoma KB 3.1 (DSM ACC 158) | 2 |
| human lung carcinoma A-549 (DSM ACC 107) | 1.5 |

## Claims

1. Pharmaceutical agent being active against multidrug resistant cell lines and comprising Epothilone A-N-oxide of the following formula:

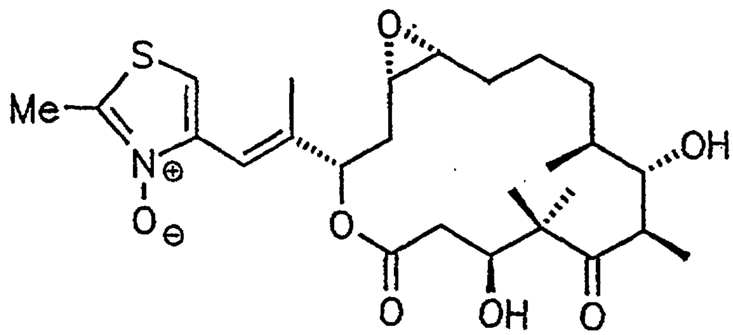

and/or Epothilone B-N-oxide of the following formula:

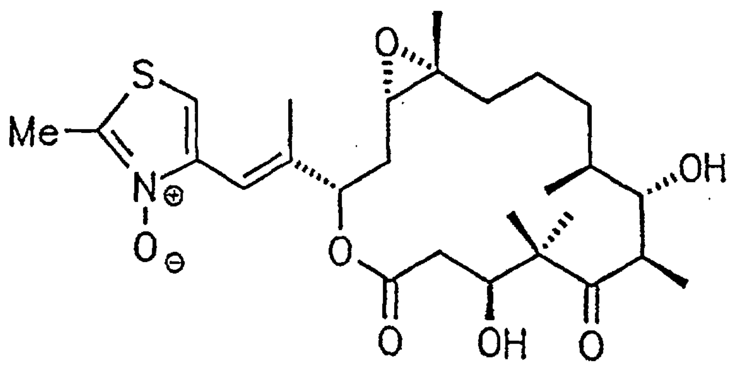

as active ingredient facultatively in the presence of usual auxiliary agents, carriers and/or diluents.

## Patentansprüche

**1.** Pharmazeutisches Mittel mit Aktivität gegen multiwirkstoffresistente Zell-Linien, umfassend Epothilon-A-N-Oxid der folgenden Formel:

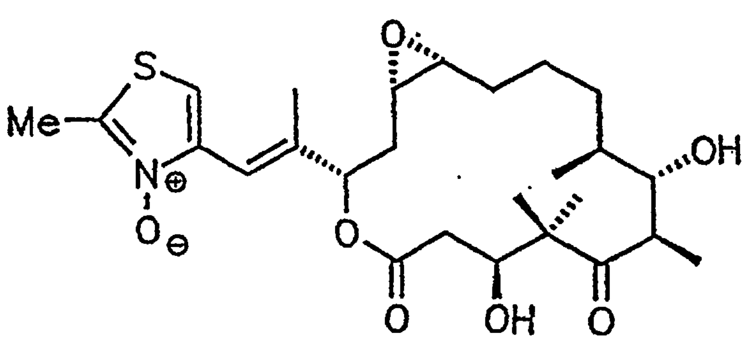

und/oder
Epothilon-B-N-Oxid der folgenden Formel:

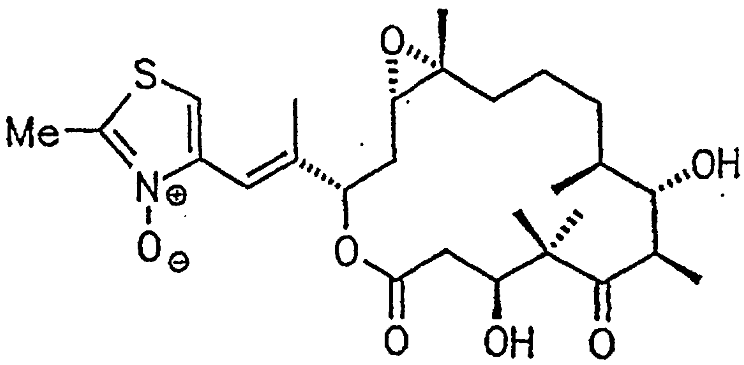

als aktiver Bestandteil gegebenenfalls in Gegenwart üblicher Hilfsmittel, Träger und/oder Verdünnungsmittel.

**Revendications**

1. Agent pharmaceutique étant actif contre les lignes de cellules résistantes à multiples médicaments et comprenant de l'oxyde A-N Epothilone avec la formule suivante:

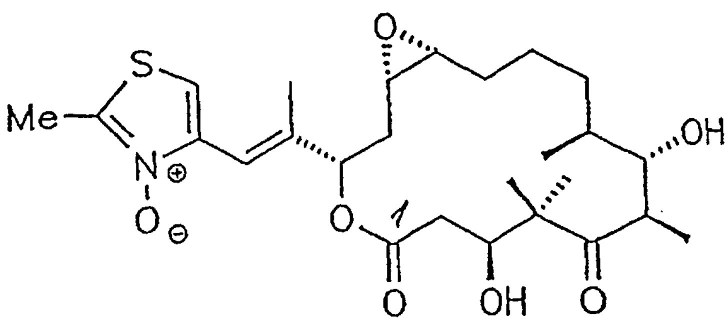

et/ou de l'oxyde B-N Epothilone avec la formule suivante:

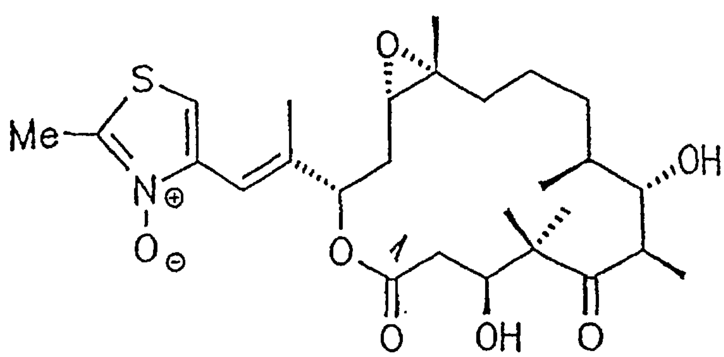

comme ingrédient actif facultatif en présence d'agents auxiliaires habituels, porteurs et/ou diluents.